Europäisches Patentamt

(19) European Patent Office

Office européen des brevets

(11) Publication number: **0 176 321**
**B1**

(12) **EUROPEAN PATENT SPECIFICATION**

(45) Date of publication of patent specification: 23.08.89

(21) Application number: 85306667.8

(22) Date of filing: 19.09.85

(51) Int. Cl.⁴: **C 12 N 15/00,** C 12 N 5/00, C 12 N 1/20 // C12R1:19, C12R1:465, C12R1:485, ·C12R1:54

(54) **Recombinant dna cosmid shuttle vectors.**

(30) Priority: 27.09.84 US 655178
07.06.85 US 742172

(43) Date of publication of application:
02.04.86 Bulletin 86/14

(45) Publication of the grant of the patent:
23.08.89 Bulletin 89/34

(84) Designated Contracting States:
BE CH DE FR GB IT LI NL SE

(56) References cited:
EP-A-0 117 694
EP-A-0 146 368

PROCEEDINGS OF THE NATIONAL ACADEMY OF SCIENCES OF THE UNITED STATES OF AMERICA, vol. 75, no. 9, September 1978, Baltimore, USA. J. COLLINS et al.: "Cosmids: A type of plasmid gene-cloning vector that is packageable in vitro in bacteriophage lambda heads", pages 4242-4246

(73) Proprietor: **ELI LILLY AND COMPANY**
**Lilly Corporate Center**
**Indianapolis Indiana 46285 (US)**

(72) Inventor: **Rao, Ramachandra Nagaraja**
**6818 Hoover Road**
**Indianapolis Indiana 46260 (US)**
Inventor: **Stanzak, Richard Kale**
**Route 2 Box 286-c**
**Poland Indiana 47868 (US)**

(74) Representative: **Hudson, Christopher Mark et al**
**Erl Wood Manor**
**Windlesham Surrey GU20 6PH (GB)**

## Description

The present invention provides novel recombinant DNA cosmid shuttle vectors comprising replicons that are functional in *Escherichia coli* and *Streptomyces*, a DNA segment contains two or more *cos* elements of bacteriophage lambda and one or more DNA segments that convey resistance to antibiotics. The invention further comprises transformants of these vectors. A method of using the cosmid shuttle vectors to construct genomic DNA libraries also is disclosed.

Cosmids are vectors specifically designed for cloning large fragments of foreign DNA. These vectors are modified plasmids which contain a plasmid replicon, a selectable drug resistance marker and the lambda *cos* site. Due to their relatively small size and the presence of the lambda *cos* element, cosmids can accept inserts of up to 30—45 kilobases (kb) and utilize the lambda *in vitro* packaging system to positively select for large size inserts. These vectors, therefore, provide an efficient mechanism to introduce foreign DNA into bacterial cells.

The present invention provides antibiotic resistance-conferring bifunctional cosmid vectors for use in *E. coli* and *Streptomyces* host cells. Bifunctional constructions are particularly advantageous because amplification and manipulation of vectors can be performed faster and more conveniently in *E. coli* than in *Streptomyces*. Thus, after desired recombinant DNA procedures are accomplished within the *E. coli* host system, the particular plasmid DNA can be removed and then transformed into a *Streptomyces* host cell. If may also be possible to transfer directly the plasmid DNA to a *Streptomyces* host cell by means of a cell-to-cell fusion or phage particle-to-cell fusion. Gene cloning and expression of products in *Streptomyces* are highly desirable because the organism is substantially non-pathogenic and ordinarily does not produce endotoxins. Heretofore, the development and exploitation of recombinant DNA technology in the above organisms has been retarded and time-consuming because of the general lack of efficient cloning systems available to accomodate large segments of DNA. The vectors of the present invention can accomodate large inserts of DNA, are functional as well as selectable in both *Streptomyces* and *E. coli* host strains and, therefore, represent a significant advance in the technical art.

The present invention further provides cosmid cloning vectors which contain multiple lambda *cos* sites. The presence of multiple *cos* sites on a single vector eliminates the need to prepare separate cosmid arms. Consequently, the construction of genomic libraries is facilitated by the structural composition of the present vectors. The present invention also provides a convenient method to construct genomic DNA libraries using the cosmid shuttle vectors provided. Presently, there are three systems available for constructing a genomic DNA library: transformation of a bacterial cell by plasmid DNA (L. Clark and J. Carbon, 1976, *Cell 9*:91); transduction of a bacterial cell by lambda bacteriophage vectors (Lawn *et al.,* 1978, *Cell 15:*1157); and transduction of a bacterial cell by cosmid vectors (Collins *et al.,* 1978, *Proc. Natl. Acad. Sci.,* 75:4242). However λ vectors are limited in their ability to accomodate DNA inserts of up to 20 kb. and large plasmids have low transformation efficiencies. Thus, the use of cosmid vectors to clone large DNA fragments is preferred over both plasmid and λ bacteriophage vectors. There is a twofold advantage of using cosmid vectors to construct genomic libraries over plasmid or lambda bacteriophage vectors. First, the ability of a cosmid vector to accomodate large DNA inserts preserves the original linkage relationships of the inserted genome. This preservation is, however, dependent on the details of the cloning procedure and is independent of the cloning system. Since larger inserts cover the entire genome, statistically, in a limited number of colonies, the screening process is thereby reduced. Secondly, the relative ease of preparation of DNA confers a beneficial advantage to the present cosmid vectors.

The specific advantage of the method according to the invention over known cosmid vectors is the ability of the present vectors to be grown and amplified in *E. coli* and then shuttled into *Streptomyces* host cells for subsequent functional analysis of the cloned DNA. The vectors are relatively small, versatile and can be transformed and selected in any *Streptomyces* cell that is sensitive to an antibiotic for which resistance is conveyed and wherein the *Streptomyces* plasmid origin of replication is functional. Because more than seventy percent of naturally occurring antibiotics are produced by *Streptomyces* strains, it is desirable to develop cloning systems and vectors that are applicable to this industrially important group. The present invention provides such vectors and thus allows for the shuttling and cloning of genes into *Streptomyces* both for increasing the yields of known antibiotics as well as for the production of new antibiotics and antibiotic derivatives.

The present invention provides vehicles for cloning DNA into *Streptomyces* host cells and also allows for the convenient selection of transformants. Since transformation is a very low frequency event, such a functional test is a practical necessity for determining which cell(s), of among billions of cells, has acquired the plasmid DNA. This is important because the foreign DNA sequences that are themselves non-selectable can be inserted into the vectors and, upon transformation, cells containing the vector and the particular DNA sequence of interest can be isolated by appropriate phenotypic selection.

For purposes of the present invention as disclosed and claimed, the following terms are defined:

Recombinant DNA Cloning Vector—any autonomously replicating agent, including but not limited to plasmids, comprising a DNA molecule to which one or more additional DNA segments can or have been added.

Cosmid—a plasmid carrying the ligated cohesive ends (cos) of bacteriophage; as a result, the plasmid DNA can be packaged either *in vitro* in the phage coat or *in vivo* using suitable *E. coli* strains.

*cos* sequence—a cohesive end site comprising a 12 nucleotide sequence from bacteriophage lambda that is recognized by the lambda-specific packaging proteins.

Library—a collection of cloned fragments of DNA, which together represent an entire genome.

Restriction Fragment—any linear DNA generated by the action of one or more restriction enzymes.

Sensitive Host Cell—a host cell that cannot grow in the presence of a given antibiotic without a DNA segment that confers resistance thereto.

Transformation—the introduction of DNA into a recipient host cell that changes the genotype and consequently results in a change in the recipient cell.

Transformant—a recipient host cell that has undergone transformation.

*E. coli* Replicon—a DNA sequence that controls and allows for replication of a plasmid or other vector in *E. coli*.

*Streptomyces* Replicon—a DNA sequence that controls and allows for replication of a plasmid or other vector in *Streptomyces*.

Ap$^R$—the ampicillin resistant phenotype.

Am$^R$—the apramycin resistant phenotype.

Tsr$^R$—the thiostrepton resistant phenotype.

Nm$^R$—the neomycin resistant phenotype.

The present invention provides a recombinant DNA cosmid shuttle vectors comprising:

a) a replicon that is functional in *E. coli*,

b) a replicon that is functional in *Streptomyces*,

c) two or more *cos* sites of bacteriophage lambda, and

d) one or more DNA segments that convey resistance to at least one antibiotic when transformed into a sensitive restrictionless host cell.

The invention further comprises transformants of the aforementioned vectors.

Vectors of the present invention represent novel hybrids between a *Streptomyces* vector and a cosmid. For example, cosmid vector pKC420 can replicate autonomously in *Streptomyces* and in *E. coli* since it contains replicons from both organisms. In addition, selectable markers are present for both organisms (Ap$^R$ in E. coli and Am$^R$ in both *E. coli* and *Streptomyces*) providing a convenient means to select transformants. Furthermore, the bacteriophage lambda *cos* sequences allow the new vector to be packaged *in vitro* and transformed into *E. coli*. The recombinant plasmids then can be used to transform *Streptomyces* host cells. Thus, given the presence of λ *cos* sequences in the present shuttle vectors, the cloning advantages inherent to cosmid vectors are now applicable to *Streptomyces*.

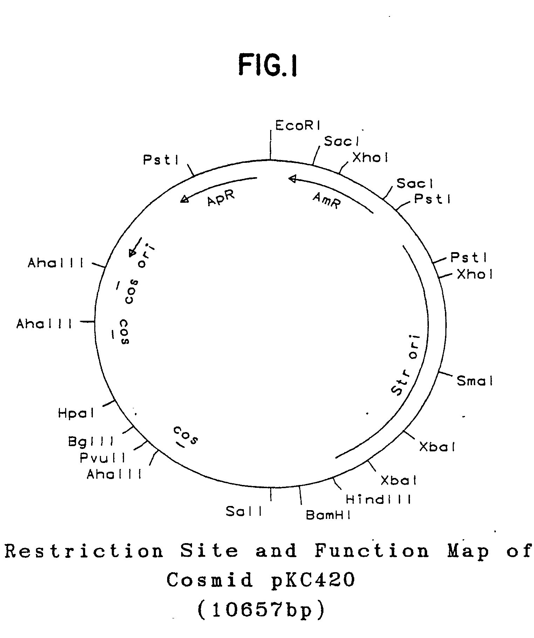

Cosmid shuttle vector pKC420 is approximately 10.6 kb and contains several restriction sites which are particularly advantageous for molecular cloning. Cosmid pKC420 can be isolated conventionally from *E. coli* K12 DH1/pKC420, a constructed strain deposited and made part of the stock culture collection of the Northern Regional Research Laboratory, Peoria, Illinois 61604. It is available to the public as a source and stock reservoir of the cosmid under the accession number NRRL B-15837. A detailed restriction site and functional map of cosmid pKC420 is presented in Fig. 1 of the accompanying drawings. For purposes of the present application, Fig. 1 and all subsequent figures are not drawn to scale.

Cosmid pKC420, useful directly as a cloning vector, also can be used to construct derivative vectors within the scope of the present invention. Cosmid pKC420 can be restricted and ligated to one or more antibiotic resistance conferring DNA fragments, exemplified herein for illustrative purposes by the thiostrepton resistance conferring ~1 kb *BCl* restriction fragment of plasmid pIJ702 (ATCC 39155), the Tn903 neomycin resistance conferring ~1.5 kb *Eco*RI restriction fragment of plasmid pUC4K (NRRL B-15836), and the Tn5 neomycin resistance conferring ~1.5 kb *Hind*III-*Sal*I restriction fragment of plasmid pKC7 (ATCC 37084), to form vectors illustrative of the present invention. Plasmids pIJ702 and pKC7 can be isolated from strains deposited and made part of the stock culture collection of the American Type Culture Collection, Rockville, Maryland 20852, and are available to the public as a source and stock reservoir of their plasmids under the accession numbers ATCC 39155 and 37084, respectively. Plasmid pUC4K, the source of the neomycin resistance conferring fragment, is a strain deposited and made part of the stock culture collection of the Northern Regional Research Laboratory, Peoria, Illinois 61604 and is available to the public as a source and stock reservoir of the plasmid under the accession number NRRL B-15836.

For convenience and ease of construction, the thiostrepton resistance conferring ~1 kb *Bcl* fragment is inserted into cosmid pKC420 at the unique *Bam*HI restriction site. The resulting recombinant DNA then is ligated to produce cosmids illustrative of the present invention. Phenotypically desired recombinant plasmids of two orientations result depending upon the orientation of the inserted DNA fragment. Thus, the insertion of the ~1 kb *Bcl* restriction fragment into cosmid pKC420 results in illustrative cosmids pKC427 and pKC427A.

Various cosmid pKC420 restriction sites can be used for the insertion of DNA segments provided that the replicons, selectable markers and other necessary plasmid functions are not disrupted. Those skilled in the art understand or can determine readily which sites are advantageous for the ligation or insertion of a particular DNA segment.

Although the thiostrepton and neomycin antibiotic resistance conferring DNA segments are, for illustrative purposes respectfully exemplified by the ~1 kb *Bcl*I restriction fragment of pIJ702, the ~1.5 kb *EcoR*I restriction fragment of plasmid pUC4K, and the ~1.5 kb *Hind*III-*Sal*I restriction fragment of plasmid pKC7, those skilled in the art can construct and substitute, either individually or in combination, other DNA segments that also confer resistance to the aforementioned antibiotics. Other thiostrepton resistance conferring DNA segments include, for example, the ~1.6 kb *BamH*I restriction fragment of plasmid pLR2. Other neomycin resistance conferring DNA segments include, for example, the ~3.5 kb *Pst*I restriction fragment and the ~3.4 kb *BamH*I restriction fragment of plasmid pLR1. Plasmids pLR2 and pLR1 are constructed in accordance with U.S. Patent No. 4,416,994 and the constructions are incorporated herein by reference.

Additional DNA segments conferring resistance to the above or to different antibiotics such as, for example, hygromycin, chloramphenicol, streptomycin, viomycin, tylosin, erythromycin, and the like also can be constructed and used for purposes of the present invention. Moreover, various functional derivatives of the above described antibiotic resistance conferring DNA segments can be constructed by adding, eliminating, or substituting nucleotides in accordance with the genetic code. Those skilled in the art will understand that ligation of these, or any other antibiotic resistance conferring DNA segments, with cosmid pKC420 DNA, results in cosmid shuttle vectors that are within the scope of the present invention.

Cosmid pKC420 and any cosmids derived therefrom, as well as the antibiotic resistance conferring DNA segments, can be conveniently modified to facilitate subsequent ligation. For example, the addition of an *EcoR*I molecular linker with *EcoR*I-*BamH*I-*EcoR*I sites to cosmid pKC427 provides for the construction of a specific restriction site, such as, for example, a *BamH*I restriction site, that is useful for ligation or for other purposes known in the art. Moreover, the various restriction fragments also can be modified by adding, eliminating, or substituting nucleotides to alter characteristics and to provide a variety of unique or additional restriction sites. Those skilled in the art understand nucleotide chemistry and the genetic code and thus which nucleotides are interchangeable and which DNA modifications are desirable for a specific purpose.

The present vectors are not limited to the use of a specific replicon from an *E. coli* or *Streptomyces* plasmid. Although the *E. coli* functional replicon exemplified in the present cosmid vectors is from plasmid pBR322, other *E. coli* replicon containing fragments can be obtained from, for example, plasmids pBR324 and pBR325 (disclosed in Bolivar, F., 1978, *Gene 4*:121), plasmid pBR328 (disclosed in Soberon, X., 1980, *Gene 9*:287), or the like to produce novel bifunctional cosmids. Additionally, other *Streptomyces* replicon containing fragments can be substituted for the *Streptomyces* replicon. These replicon containing fragments include, but are not limited to, replicons from plasmids SCP2 and SCP2* (disclosed in Bibb and Hopwood, 1981, *J. Gen. Microbiol. 126:*427), SLP1 (disclosed in Bibb, M. J., 1981, *Mol. Gen. Genet. 184*:230), pEL103 (NRRL 12549), pFJ265 (disclosed in Jones, M. D., *et al.*, 1984, *Plasmid 11*:92) and pHJL210 (NRRL B-15824). Those skilled in the art will understand that ligation of these, or other *E. coli* or *Streptomyces* replicon containing fragments, results in cosmid shuttle vectors that are within the scope of the present invention.

The recombinant DNA cosmid shuttle vectors of the present invention are not limited for use in a single species or strain of Streptomyces. To the contrary, the vectors are broadly applicable and can be transformed into host cells of many *Streptomyces* taxa, particularly restrictionless strains of economically important taxa that produce antibiotics such as aminoglycoside, macrolide, β-lactam, polyether and glycopeptide antibiotics. Such restrictionless strains are selected and isolated readily from *Streptomyces* taxa by conventional procedures well known in the art. (Lomovskaya *et al.*, 1980, Microbiological Reviews 44:206). Host cells of restrictionless strains lack restriction enzymes and, therefore, do not cut or degrade plasmid DNA upon transformation. For purposes of the present application, host cells containing restriction enzymes that do not cut any of the restriction sites of the present recombinant cosmids also are considered restrictionless.

Preferred host cells of restrictionless strains of *Streptomyces* taxa that produce aminoglycoside antibiotics and in which the present vectors are especially useful and can be transformed, include restrictionless cells of, for example: *Streptomyces* kanamyceticus (kanamycins), *S. chrestomyceticus* (aminosidine), *S. griseoflavus* (antibiotic MA 1267), *S. microsporeus* (antibiotic SF-767), *S. ribosidificus* (antibiotic SF733), *S. flavopersicus* (spectinomycin), *S. spectabilis* (actinospectacin), *S. rimosus* forma *paromomycinus* (paromomycins, catenulin), *S. fradiae* var. *italicus* (aminosidine), *S. bluensis* var. *bluensis* (bluensomycin), *S. catenulae* (catenulin), *S. olivoreticuli* var. *cellulophilus* (destomycin A), *S. lavendulae* (neomycin), *S. albogriseolus* (neomycins), *S. ablus* var. *metamycinus* (metamycin), *S. hygroscopicus* var. *sagamiensis* (spectinomycin), *S. bikiniensis* (streptomycin), *S. griseus* (streptomycin), *S. erythrochromogenes* var. *narutoensis* (streptomycin), *S. poolensis* (streptomycin), *S. galbus* (streptomycin) *S. rameus* (streptomycin), *S. olivaceus* (streptomycin) *S. mashuensis* (streptomycin), *S. hygroscopicus* var. *limoneus* (validamycins), *S. rimofaciens* (destomycins), *S. hygroscopicus* forma *glebosus* (glebomycin) *S. fradiae* (hybrimycins neomycins), *S. eurocidicus* (antibiotic A16316-C), *S. aquacanus* (N-methyl hygromycin B), *S. crystallinus* (hygromycin A). *S. noboritoensis* (hygromycin) *S. hygroscopicus* (hygromycins), *S. atrofaciens* (hygromycin), *S. kasugaspinus* (kasugamycins), *S. kasugaensis* (kasugamycins), *S. netropsis* (antibiotic LL-AM31), *S. lividus* (lividomycins), *S. hofuensis*

(seldomycin complex), and *S. canus* (ribosyl paromamine).

Preferred host cells of restrictionless strains of *Streptomyces* taxa that produce macrolide antibiotics and in which the present vectors are especially useful and can be transformed, include restrictionless cells of, for example: *Streptomyces caelestis* (antibiotic M188), *S. platensis* (platenomycin), *S. rochei* var. *volubilis* (antibiotic T2636), *S. venezuelae* (methymycins), *S. narbonensis* (josamycin, narbomycin), *S. fungicidicus* (antibiotic NA-181), *S. griseofaciens* (antibiotic PA133A, B), *S. roseocitreus* (albocycline), *S. bruneogriseus* (albocycline), *S. roseochromo genes* (albocycline), *S. cinerochromogenes* (cineromycin B), *S. albus* (albomycetin), *S. felleus* (argomycin, picromycin), *S. rochei* (lankacidin, borrelidin), *S. violaceoniger* (lankacidin), *S. griseus* (borrelidin), *S. maizeus* (ingramycin), *S. albus* var. *colimyceticus* (coleimycin), *S. mycarofaciens* (acetyl-leukomycin, espinomycin) *S. hygroscopicus* (turimycin, relomycin, maridomycin, tylosin, carbomycin), *S. griseospiralis* (relomycin), *S. lavendulae* (aldgamycin), *S. rimosus* (neutramycin), *S. deltae* (deltamycins), *S. Fungicidicus* var. *espinomyceticus* (espinomycins), *S. furdicidicus* (mydecamycin), *S. ambofaciens* (foromacidin D), *S. eurocidicus* (methymycin), *S. griseolus* (griseomycin), *S. flavochromogenes* (amaromycin, shincomycins) *S. fimbriatus* (amaromycin), *S. fasciculus* (amaromycin), *S. erythreus* (erythromycins), *S. antibioticus* (oleandomycin), *S. olivochromogenes* (oleandomycin), *S. spinichromogenes* var. *suragaoensis* (kujimycins), *S. kitasatoensis* (leucomycin), *S. narbonensis* var. *josamyceticus* (leucomycin A3, josamycin), *S. albogriseolus* (mikonomycin), *S. bikiniensis* (chalcomycin), *S. cirratus* (cirramycin), *S. djakartensis* (niddamycin), *S. eurythermus* (angolamycin), *S. fradiae* (tylosin, lactenocin, macrocin), *S. goshikiensis* (bandamycin), *S. griseoflavus* (acumycin), *S. halstedii* (carbomycin), *S. tendae* (carbomycin), *S. macrosporeus* (carbomycin), *S. thermotolerans* (carbomycin), and *S. albireticuli* (carbomycin).

Preferred host cells of restrictionless strains of *Streptomyces* taxa that produce β-lactam antibiotics and in which the present vectors are especially useful and can be transformed, include restrictionless cells of, for example: *Streptomyces lipmanii* (A16884, MM4550, MM13902), *S. clavuligerus* (A16886B, clavulanic acid), *S. lactamdurans* (cephamycin C), *S. griseus* (cephamycin A, B), *S. hygroscopicus* (deacetoxycephalosporin C), *S. wadayamensis* (WS-3442-D), *S. chartreusis* (SF1623), *S. heteromorphus* and *S. panayensis* (C2081X); *S. cinnamonensis, S. fimbriatus, S. halstedii, S. rochei* and *S. viridochromogenes* (cephamycins A, B); *S. cattleya* (thienamycin); and *S. olivaceus, S. flavovirens, S. flavus, S. fulvoviridis, S. argenteolus,* and *S. sioyaensis* (MM 4550 and MM 13902).

Preferred host cells of restrictionless strains of *Streptomyces* taxa that produce polyether antibiotics and in which the present vectors are especially useful and can be transformed, include restrictionless cells of, for example: *Streptomyces albus* (A204, A28695A and B, salinomycin), *S. hygroscopicus* (A218, emericid, DE3936), A120A, A28695A and B, etheromycin, dianemycin), *S. griseus* (grisorixin), *S. conglobatus* (ionomycin), *S. eurocidicus* var. *asterocidicus* (laidlomycin), *S. lasaliensis* (lasalocid), *S. ribosidificus* (lonomycin), *S. cacaoi* var. *asoensis* (lysocellin), *S. cinnamonensis* (monensin), *S. aureofaciens* (narasin), *S. gallinarius* (RP30504), *S. longwoodensis* (lysocellin), *S. flaveolus* (CP38936), *S. mutabilis* (S-11743a), and *S. violaceoniger* (nigericin).

Preferred host cells of restrictionless strains of *Streptomyces* taxa that produce glycopeptide antibiotics and in which the present vectors are especially useful and can be transformed, include restrictionless cells of, for example: *Streptomyces orientalis* and *S. haranomachiensis* (vancomycin); *S. candidus* (A-35512, avoparcin) *S. eburosporeus* (LL-AM 374), and *S. toyocaensis* (A47934).

Preferred host cells of other *Streptomyces* restrictionless strains in which the present vectors are specially useful and can be transformed, include restrictionless cells of, for example: *Streptomyces coelicolor, S. granuloruber, S. roseosporus, S. lividans, S. tenebrarius, S. acrimycins, S. glaucescens, S. parvilin, S. pristinaespiralis, S. violaceoruber, S. vinaceus, S. virginiae, S. espinosus,* and *S. azureus.*

In addition to the representative *Streptomyces* host cells described above, the present vectors are also useful and can be transformed into cells of restrictionless strains of other taxa such as, for example: *Bacillus, Staphylococcus* and related Actinomycetes, including *Streptosporangium, Actinoplanes, Nocardia,* and *Micromonospora.* Thus, the vectors of the present invention have wide application, are useful in and can be transformed into host cells of a variety of organisms.

While all the embodiments of the present invention are useful, some of the present recombinant DNA cloning vectors and transformants are preferred. Accordingly, preferred vectors are cosmids pKC420, pKC427, pKC428, pKC448, pKC462A, and pKC467; and preferred transformants are *Streptomyces ambofaciens*/pKC420, *S. ambofaciens*/pKC427, *S. ambofaciens*/pKC428, *S. ambofaciens*/pKC448, *S. ambofaciens*/pKC462A, *S. ambofaciens*/pKC467, *E. coli* K12 SF8/pKC420, *E. coli* K12 SF8/pKC427, *E. coli* K12 SF8/pKC428, *E. coli* K12 SF8/pKC448, *E. coli* K12 SF8/pKC462A and *E. coli* K12 SF8/pKC467. Moreover, of this preferred group, cosmids pCK420, pKC462A, and pKC467, and transformants *S. ambofaciens*/pKC420, *S. ambofaciens*/pKC462A, *S. ambofaciens*/pKC467, *E. coli* K12 SF8/pKC420, *E. coli* K12 SF8/pKC462A, and *E. coli* K12 SF8/pKC467 are most preferred.

The recombinant DNA cloning vectors and transformants of the present invention have broad utility and help fill the need for suitable cloning vehicles for use in *Streptomyces* and related organisms. Moreover, the ability of the present vectors to confer resistance to antibiotics that are toxic to non-transformed host cells, also provides a functional means for selecting transformants. This is important because of the practical necessity for determining and selecting the particular cells that have acquired vector DNA. Additional DNA segments, that lack functional tests for their presence, can also be inserted in the present vectors and then transformants containing the non-selectable DNA can be isolated by appropriate antibiotic selection. Such non-selectable DNA segments can be inserted at any site, except within regions necessary for plasmid function, maintainance, and replication and include, but are not limited to, genes that specify antibiotic modification enzymes, antibiotic resistance, antibiotic biosynthesis, and regulatory genes of all types.

The present invention further comprises a novel method for using the aforedefined recombinant DNA cosmid shuttle vectors to construct a genomic DNA library, said method comprising:

a) ligating a genomic DNA segment into a cosmid shuttle vector, as defined earlier;

b) packaging said ligated cosmid into bacteriophage lambda particles,

c) transducing said packaged cosmid into *E. coli*, and

d) transforming the recombinant cosmid into a *Streptomyces* host cell.

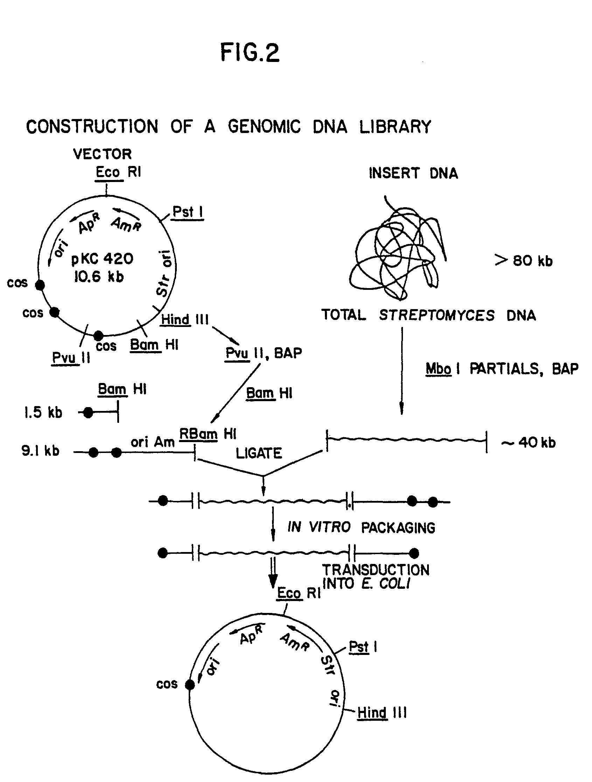

More particularly and with reference to Figure 2, cosmid pKC420 DNA was digested with *Pvu*II restriction enzyme to generate a linear fragment with blunt ends. These blunt ends were then dephosphorylated with bacterial alkaline phosphatase (BAP) to prevent their ligation in subsequent reactions. After extraction and precipitation, the DNA was digested with *Bam*HI restriction enzyme to generate two DNA fragments of unequal length, each fragment consisting of a *cos* site flanked by a reactive *Bam*HI end and a non-reactive *Pvu*II end. The DNA was extracted, precipitated and then dissolved in TE buffer for subsequent ligation to the insert DNA fragments. Although the preferred embodiment of the method of this invention employs cosmid pKC420, it is apparent that any one of the following cosmids may be used to construct genomic libraries: pKC427, pKC428, pKC448, pKC462, pKC462A, or pKC467.

Foreign DNA, such as, for example, *Streptomyces felleus* DNA (NRRL 2251) was partially digested with a restriction enzyme, such as *Mbo*I or *Sau*3A, to generate a size range (average size of 40 kb) of *S. felleus* DNA fragments. These fragments subsequently were treated with BAP to prevent any *Mbo*I-generated ends from ligating to each other. Thus, the only allowed ligation was between the *Mbo*I ends of the insert DNA and the compatible *Bam*HI ends of the cosmid DNA. The resultant hybrid DNA molecules served as substrates for *in vitro* packaging of bacteriophage lambda particles. Due to the size selection mechanism of lambda packaging, only those cosmid-insert-DNA molecules in which the *cos* sequences are 37 to 52 kb apart were packaged. Because lambda packaging required two *cos* sites, one to initiate and another to terminate packaging and is also size selective, large inserts were positively selected in the transductants. After the desired recombinant DNA procedures were accomplished within the *E. coli* host system, the particular recombinant plasmid DNA was isolated and then transformed into a suitable *Streptomyces* host.

The method of the present invention enhances both the efficiency and effectiveness of cosmid cloning given the specific construction of these novel cosmid vectors. For example, the presence of two or more *cos* sites in cosmid pKC420 eliminates the need to prepare separately two cosmid arms. Secondly, the presence of blunt-end restriction enzyme sites such as the *Pvu*II and *Hpa*I sites within the multiple *cos* site region prevents subsequent cosmid concatemerization between the prepared cosmid arms. Ultimately, given the bifunctionality of the present vectors, the cloned DNA can be shuttled into a *Streptomyces* host cell for functional analysis of the cloned DNA. This shuttling capability is particularly advantageous over known cosmid vehicles because *Streptomyces* cosmid vectors have yet to be exploited.

*Escherichia coli* K12 DH1/pKC420, as a source of cosmid vector pKC420 (NRRL B-15837) and *E. coli* K12 SF8/pKC462A, as a source of cosmid vector pKC462A (NRRL B-15973), can be cultured in a number of ways using any of several different media. Carbohydrate sources which are preferred in a culture medium include, for example, glucose and glycerol, and nitrogen sources include, for example, ammonium salts, amino acid mixtures, and peptones. Nutrient inorganic salts also are incorporated and include the customary salts capable of yielding magnesium, sodium, potassium, ammonia, calcium, phosphate, chloride, sulfate, and like ions. As is necessary for the growth and development of other microorganisms, essential trace elements also are added. Such trace elements commonly are supplied as impurities incidental to the addition of other constituents of the medium.

*E. coli* K12 DH1/pKC420 and *E. coli* K12 SF8/pKC462A were grown under aerobic culture conditions over a relatively wide pH range of about 6.5 to 7.4 at temperatures ranging from about 30° to 42°C. For the production of the cosmid vectors pKC420 and pKC462A in the greatest quantity, however, it is desirable to start with a culture medium at a pH of about 7.4 and maintain a culture temperature of about 37°C. Culturing the *E. coli* cells under the aforementioned conditions resulted in a reservoir of cells from which cosmids pKC420 and pKC462A were isolated by techniques well known in the art.

Brief description of the figures

Fig. 1 shows the restriction site and function map of cosmid pKC420.

Fig. 2 is a schematic outline of the method of constructing genomic DNA libraries using cosmid vector pKC420 and *Streptomyces* DNA.

Fig. 3 is a restriction site and function map of cosmid pKC427.

Fig. 4 is a restriction site and function map of cosmid pKC428.

Fig. 5 is a restriction site and function map of cosmid pKC448.

Fig. 6 is a restriction site and function map of cosmid pKC462.

Fig. 7 is a restriction site and function map of cosmid pKC467.

Fig. 8 is a restriction site and function map of cosmid pKC462A.

Fig. 9 is a restriction site and function map of plasmid pOJ108.

Fig. 10 is a restriction site and function map of plasmid pOJ111.

Fig. 11 is a restriction site and function map of cosmid cos111.

The following non-limiting examples further illustrate and detail the invention disclosed. Both an explanation of the actual procedures for constructing the invention are described where appropriate.

Example 1
Culture of *E. coli* K12 DHI/pKC420 and isolation of cosmid pKC420

A. Culture
5 ml. cultures of *E. coli* K12 DH1/pKC420 (NRRL B-15837) were grown under selective conditions in TY media (1% tryptone, .5% yeast extract, .5% sodium chloride, pH 7.4) according to conventional microbiological procedures. The cells were spun in a table top centrifuge and the pellet resuspended in 1 ml. of 0.3M sucrose, 25 mM EDTA (ethylenediaminetetracetic acid) and 25 mM Tris-HCl pH 8 (Solution I). After transfer to an Eppendorf tube the cells were centrifuged for about one minute and the pellet was resuspended in 0.5 ml. of Solution I. About 50 µl. of freshly made lysozyme (20 mg./ml. in water) was added and the solution was incubated for 10 minutes at 37°C.

After the addition of 250 µl. of freshly made lysis mix (2% sodium dodecyl sulfate and 0.3N NaOH), the cells were immediately and completely vortexed. The cells were then incubated for ten minutes at 50°C., cooled and added to 100 µl. of phenol-Sevag (phenol-chloroform-isoamyl alcohol, 25-24-1). After the DNA was centrifuged for two minutes in an Eppendorf centrifuge, the supernatant was decanted and transferred to another tube with 70 µl. of unbuffered 3M sodium acetate and isopropanol to precipitate the DNA. This solution was incubated for five minutes at room temperature and then centrifuged for two minutes. The supernatant was gently and completely decanted to remove all the excess liquid.

The DNA precipitate was redissolved in 500 µl. of TE (10 mM Tris-HCl pH 8 and 1 mM EDTA) and 10 µl. of 100 mM Spermine HCl was added. This

mixture was vortexed and then incubated for five minutes at room temperature before a five minute spin in an Eppendorf centrifuge. The supernatant was again completely decanted and discarded and the precipitated DNA was vortexed with 1 ml. of 75% ethanol, 0.3M sodium acetate, and 10 mM magnesium acetate. This solution was incubated for five minutes at room temperature and the DNA collected as above. The pellet was redissolved in 10 µl. of TE for subsequent use as a cloning vehicle.

Example 2
Construction of cosmid shuttle vector pKC427

A. *Bam*HI digestion of cosmid pKC420
About 5 µg of cosmid pKC420 were digested in 1× *Bam*HI buffer (150 mM NaCl, 6 mM Tris-HCl pH 7.9, 6 mM MgCl$_2$ and 1 mM Dithiothreitol) in a total volume of 50 µl. with 20 units (New England Biolab) of *Bam*HI restriction endonuclease*. The mixture was incubated at 37°C. for about 1 hour and then the reaction was terminated by incubation at 70°C. for 10 minutes. Since cosmid pKC420 has a single *Bam*HI site, digestion is easily monitored by agarose gel electrophoresis. The appearance of a single band of about 10 kb signals complete digestion. The DNA was extracted with phenol and Sevag (chloroform:isoamyl alcohol 24:1), precipitated with ethanol, dried and then resuspended in TE for subsequent ligation.

B. *Bcl*l digestion of plasmid pIJ702
About 5 µg of plasmid pIJ702 DNA (ATCC 39155) were digested in 1× *Bcl*l buffer (75 mM KCl, 6 mM Tris-HCl pH 7.4, 10 mM MgCl$_2$ and 1 mM Dithiothreitol) in a total volume of 50 µl. with 10 units (New England Biolab) of *Bcl*l restriction endonuclease. The mixture was incubated at 50°C. for about an hour and then the reaction was terminated by extraction with phenol and Sevag, precipitated with ethanol, dried and then dissolved in 5 µl. TE. The DNA was electrophoresed on a 0.5% agarose gel until the desired ~1 kb *Bcl*l fragment was separated from other fragments. Whatman DEAE cellulose paper was placed in a slit prepared ahead of the desired DNA band and the DNA was electrophoresed onto the DEAE paper. The paper was washed with 1 ml. of TE and the DNA was eluted with 400 µl. of TE adjusted to 1M by the addition of an appropriate volume of NaCl. The eluted DNA was ethanol precipitated and finally dissolved in 5 µl. of TE.

*Restriction enzymes and instructions can be obtained from the following sources:
New England Bio Labs., Inc., 32 Tozer Road, Beverly, Massachusetts 01915
Bethesda Research Laboratories, Inc., P.O. Box 577, Gaithersburg, Maryland 20760
Boehringer-Mannheim Biochemicals, P.O. Box 50816, Indianapolis, Indiana 46250 C. Ligation and construction of *E. coli* K12 SF8/ pKC427

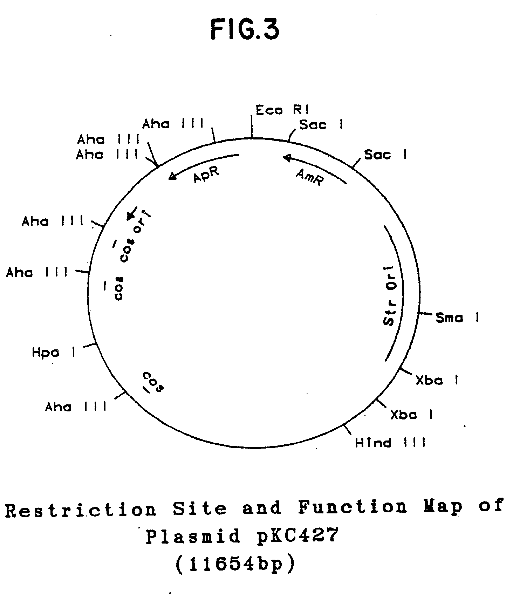

About 1 µg. each of *Bam*HI-digested cosmid pKC420 DNA and the ~1 kb *Bcl*I thiostrepton resistance-conferring fragment were ligated in 20 µl. of 1× ligase buffer (50 mM Tris-HCl pH 7.8, 10 mM $MgCl_2$, 20 mM Dithiothreitol and 1 mM ATP) with 400 units of T4 DNA ligase* for 16 hours at 16°C. The reaction was terminated by incubation at 70°C. for 10 minutes. After cooling on ice, the resultant ligated DNA was used to transform *E. coli* K12 SF8 (NRRL B-15835) according to the procedure of Maniatis *et al.*, 1982, Molecular Cloning, Cold Spring Harbor Laboratory, Cold Spring Harbor, New York. The identity of the desired transformants was conventional confirmed by screening for the loss of the *Bam*HI site and the acquisition of a *Sal*I site. Competent cells were stored in 20% glycerol, instead of dimethyl sulfoxide, at −70°C. The resultant *E. coli* K12 SF8/ pKC427 transformants were conventionally cultured for subsequent production and isolation of cosmid pKC427. A restriction site and function map of cosmid pKC427 is presented in Figure 3 of the accompanying drawings.

Example 3
Construction of cosmid shuttel vector pKC428

A. *Eco*Ri digestion of cosmid pKC427

About 5 µg. of cosmid pKC427 DNA were digested in 1× *Eco*RI buffer (50 mM NaCl, 100 mM Tris-HCl pH 7.5, 5 mM $MgCl_2$ and 1 mM Dithiothreitol) in a total volume of 50 µl. with 20 units (New England Biolab) of *Eco*RI restriction endonuclease. The mixture was incubated at 37°C for about 1 hour and then the reaction was terminated by incubation at 70°C. for 10 minutes. Since cosmid pKC427 contains a unique *Eco*RI site, an *Eco*RI digestion generates a single linear fragment.

B. *Eco*RI digestion of plasmid pUC4K and isolation of the ~1.5 kb *Eco*RI neomycin resistance-conferring gene

The desired digestion was carried out in substantial accordance with the teaching of Example 3A except that plasmid pUC4K (NRRL B-15836) DNA, rather than cosmid pKC427 DNA, was used. The isolation of the ~1.5 kb *Eco*RI fragment was carried out in substantial accordance with the teaching of Example 2B.

C. Ligation and construction of *E. coli* K12 SF8/ pKC428

The ligation and subsequent transformation procedures were carried out in substantial accordance with the teaching of Example 2C. The identity of the desired transformants was conventionally confirmed by initially selecting for Am[R]

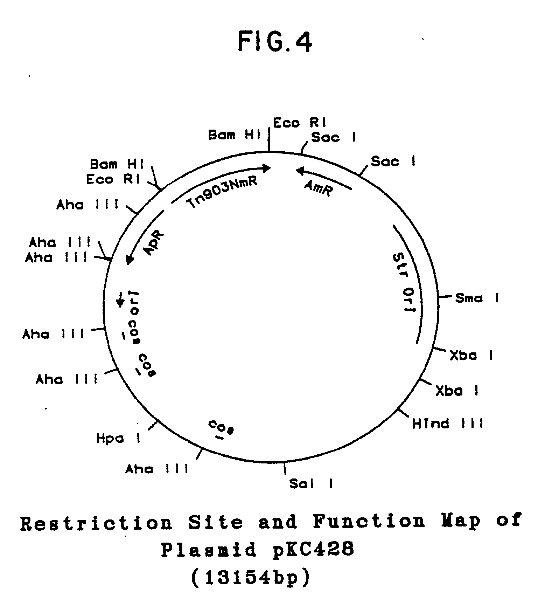

phenotype and then replicating those Am[R] colonies to select for neomycin resistant colonies. These colonies were additionally screened for the acquisition of *Bam*HI and *Sal*I restriction sites. A restriction site and function map of cosmid pKC428 is presented in Figure 4 of the accompanying drawings.

Example 4
Construction of cosmid shuttle vector pKC448

A. *Bam*HI digestion of cosmid pKC428 and subsequent ligation

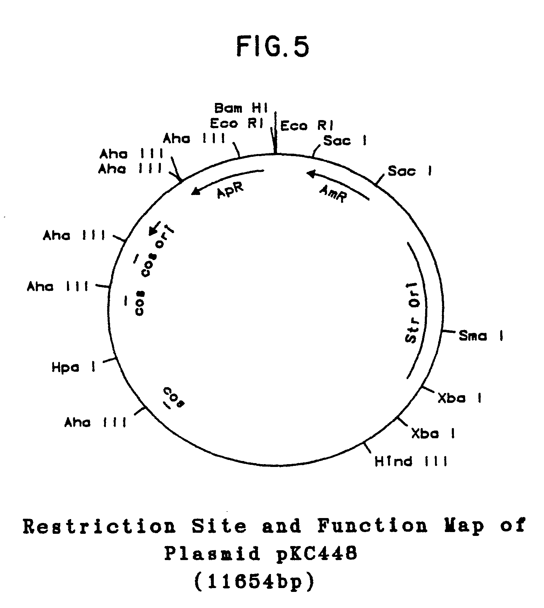

Cosmid pKC448 was constructed by deleting a *Bam*HI fragment from cosmid pKC428. The desired *Bam*HI digestion was carried out in substantial accordance with the teaching of Example 2A except that cosmid pKC428 DNA was used in place of cosmid pKC420 DNA. The resulting fragments were recovered and self-ligated in substantial accordance with the teaching of Examples 2B and 2C. This digestion results in the removal of the neomycin resistance conferring gene and generates a unique *Bam*HI site flanked by two *Eco*RI sites. A restriction site and function map of cosmid pKC448 is presented in Figure 5 of the accompanying drawings.

Example 5
Construction of cosmid pKC462

A. *Hind*III and *Sal*I digestion of cosmid pKC448

About 10 µg. of cosmid pKC448 were digested in 100 µl. of buffer (150 mM NaCl, 6 mM Tris-HCl pH 7.9, 6 mM $MgCl_2$ and 1 mM Dithiothreitol) with 20 units each of *Hind*III and *Sal*I restriction enzymes for 2 hours at 37°C. The DNA was ethanol precipitated and then resuspended in 20 µl. of TE.

B. Digestion of plasmid pKC7 DNA and isolation of the neomycin resistance-conferring gene

The desired digestion and isolation of the ~1.5 kb *Hind*III-*Sal*I fragment is carried out in substantial accordance with the teaching of Example 5A except that plasmid pKC7 (ATCC 37084) is used in place of cosmid pKC448.

C. Ligation and construction of *E. coli* DH1/ pKC462

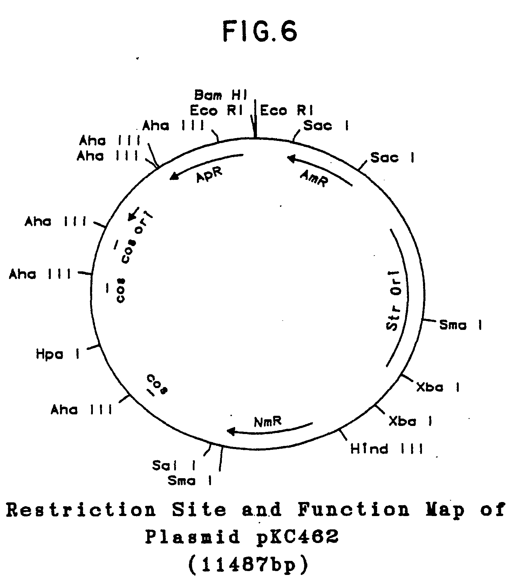

The ligation and subsequent transformation procedures were carried out in substantial accordance with the teaching of Example 2C except that *E. coli* DH1 (NRRL B-15021) was used in place of *E. coli* K12 SF8. In addition, cosmid pKC462 DNA was used in place of cosmid pKC427 DNA. The identity of the desired transformants was conventionally confirmed by initially selecting for Am[R] phenotype and then replicating those Am[R] colonies to select for neomycin resistant colonies. A restriction site and function map of cosmid pKC462 is presented in Figure 6 of the accompanying drawings.

*T4 DNA ligase can be obtained from the same sources as those identified for restriction enzymes.

Example 6
Construction of *E. coli* K12 SF8/pKC448

The desired construction was made, selected, and recovered in substantial accordance with the teaching of Example 2C except that cosmid pKC448 DNA was used in place of cosmid pKC427 DNA. The identified transformants were then used for subsequent production and isolation of cosmid pKC448 according to the teaching of Example 1.

Example 7
Construction of *Streptomyces ambofaciens*/pKC420 and *S. ambofaciens*/pKC448

About 1 µg. each of the DNA from Examples 1 and 6 and 200 µl. of protoplasts of *Streptomyces ambofaciens*, a strain deposited and made part of the permanent stock culture collection of the Northern Regional Research Laboratories, Peoria, Illinois, from which it is available to the public under the accession number NRRL 2420, were mixed with 500 µl. of 55% polyethylene glycol (Sigma) in P medium (Hopwood and Wright, 1978, Molecular and General Genetics 162:307), vortexed and then aliquots of 25 µl. and 250 µl. were plated onto R2YE* plates with 3 ml. of R2YE top agar. The plates were incubated for 18 hours at 30°C. and then overlayed with 3 ml. of R2YE top agar containing sufficient apramycin** for a final concentration of 50 µg./ml. The plates were then incubated for an additional 3 days at 30°C. The resultant *S. ambofaciens*/pKC420 and *S. ambofaciens*/pKC448 apramycin resistant colonies were isolated according to known procedures, cultured, and then conventionally identified by restriction enzyme and agarose gel electrophoretic analysis of their constitutive cosmids (Maniatis *et al.*, 1982).

Example 8
The construction of a genomic library

A. Preparation of the vector pKC420 DNA

About 50 µg. of cosmid pKC420 DNA were digested in 500 µl. of 1× *Pvu*II (60 mM NaCl, 6 mM Tris-HCl pH 7.5, 6 mM MgCl₂ and 1 mM Dithiothreitol) buffer with 100 units of *Pvu*II restriction enzyme for 3 hours at 37°C. About 50 µl. of 10× BAP buffer (500 mM Tris-HCl pH 8 and 500 mM NaCl) and 2.5 units of BAP (International Biotechnologies, Inc., P.O. Box 1565, New Haven, CT 06506) were added and incubated for 1 hour at

70°C. The DNA was extracted with phenol, Sevag and precipitated with ethanol The DNA was then digested in 500 µl. of 1× *Bam*HI buffer with 90 units of *Bam*HI restriction enzyme for 3 hours at 37°C. The DNA was again extracted with phenol, Sevag, precipitated with ethanol and finally dissolved in 50 µl. of TE.

B. Preparation of the insert DNA

*Streptomyces felleus* (NRRL 2251) was grown in 250 ml. of Tryptic Soy broth supplemented with 100 µg./ml. of spiramycin* for 16 hours at 30°C. The cells were harvested by centrifugation (10 minutes at 8,000 rpm) suspended in 10 ml. of lysis mix (300 mM Sucrose, 25 mM Tris-HCl pH 8, and 25 mM EDTA) and brought to a final concentration of 1 mg./ml. with lyzozyme and incubated at 37°C. for 10 minutes. The proteinase K was added to a final concentration of 200 µg./ml. and SDS (sodium dodecyl sulfate) was added to a final concentration of 2%. The mixture was incubated at 70°C. for 10 minutes and then cooled on ice. The mixture was made 1M in potassium acetate and left on ice for 30 minutes. After gently extracting the material with TE saturated phenol, the layers were separated and the aqueous layer was gently extracted with Sevag. Layers were again separated and the nucleic acids in the aqueous layer were precipitated with ethanol. The precipitate was washed with 70% ethanol and then dissolved in 5 ml. TE. RNase A was added to the DNA solution to a final concentration of 50 µg./ml. This solution was then incubated at 37°C. for 30 minutes, extracted twice with phenol, twice with Sevag and then precipitated with ethanol. The DNA was redissolved in 1 ml. TE (545 µg./ml.) and then sized on a 0.3% agarose gel with λ standards and was found to have an average size of 70 kb.

Next, 50 µg. of *Streptomyces felleus* chromosomal DNA were incubated with 30 units of *Mbo*I in 500 µl. of 1× *Mbo*I buffer (100 mM NaCl, 10 mM Tris-HCl pH 7.4, 10 mM MgCl₂, and 1 mM DTT) at 37°C. for 15 minutes. This particular condition was found, empirically, to give the desired partial fragmentation of chromosomal DNA. The DNA was extracted with phenol, Sevag and then dissolved in 50 µl. of TE.

About 25 µg. of *Streptomyces felleus Mbo*I partials were subsequently treated with BAP (1.25 units for the first 1 hour at 70°C. and then an additional 1.25 units for another hour at 70°C.) in 100 µl of 1× BAP buffer. The DNA was extracted with phenol, Sevag, precipitated with ethanol and then dissolved in 50 µl. of TE. The size of this DNA was estimated on a 0.3% agarose gel and was found to be 30—40 kb.

C. Ligation of the vector DNA to the insert DNA

About 125 ng. of the pKC420 arms (prepared in Example 8A) were mixed with 500 ng. of *Strep-*

---

*R2YE medium was prepared with the following composition per liter:
    Sucrose—103 g. 2.5% K₂SO₄—10 ml. MgCl₂—10.1 g. Glucose—10 g. Casamino acids—0.1 g. Agar—22 g. Trace Element Mix—2 ml. 0.5% KH₂PO₄—10 ml. 1M CaCl₂—20 ml. Proline—3 g. 0.25M TES pH 7.2—100 ml. 10% Yeast extract—50 ml.
**Antibiotic apramycin can be obtained from either Sigma, St. Louis, Missouri or Eli Lilly and Company, Indianapolis, Indiana.

---

*Antibiotic spiramycin can be obtained from Sigma, St. Louis, Missouri.

*tomyces felleus* Mbol partials (prepared in Example 8B) and ligated with 400 units (New England Biolabs) of T4 DNA ligase in 20 μl. of 1× ligase buffer made 1 mM in ATP. Ligation was performed for 16 hours at 16°C. and then terminated by heating for 10 minutes at 70°C.

D. In vitro packaging

Packaging was performed by adding about 10 μl. of the ligation mixture (~62.5 ng of hybrid vector DNA) to Biotec packaging kit* at 30°C. for 1 hour. To this mixture, about 500 μl. of 0.1M NaCL, 0.1M Tris-HCl pH 8, and 0.01M $MgSO_4$ were added. Lastly, 25 μl. of chloroform were added to kill any living bacteria.

E. Transduction of *E. coli* K12 SF8

About 200 μl. of packaged cosmids (25 ng of vector DNA) were adsorbed to 500 μl. of *E. coli* strain K12 SF8 grown in Tryptone yeast extract supplemented with 0.2% maltose and 10 mM magnesium sulfate. Adsorption was performed for 10 minutes at 37°C in 10 mM Tris pH 8.0 and 10 mM $MgSO_4$. The cells were grown in 5 ml. of Tryptone yeast extract for three hours at room temperature and transductants were selected at 30°C. on plates supplemented with 200 μg./ml. of apramycin. Approximately 400 colonies resulted from the plating of 0.1 ml. of transduced cells giving rise to a transducing efficiency of about $1.2×10^6$ transductants per microgram.

F. Transformation into *Streptomyces ambofaciens*

The desired transformation was performed in substantial accordance with the teaching of Example 7. About $3.4×10^4$ transformants per 1 μg. of *E. coli* grown pKC420 were obtained in this experiment.

Example 9
Construction of cosmid pKC467

A. *XbaI* digestion of cosmid pKC462

About 25 μg of cosmid pKC462 are digested with 200 units of *XbaI* restriction enzyme in 100 μl. of IX *XbaI* buffer (50 mM NaCl, 100 mM Tris-HCl pH 7.5, 5 mM $MgCl_2$ and 1 mM Dithiothreitol). The mixture is incubated at 37°C. for about 1 hour and then the reaction is terminated by incubation at 70°C. for 10 minutes. The digested DNA is electrophoresed on a 0.5% agarose gel (International Biotech, Inc.) and then the large fragment is isolated on DEAE paper. The isolated DNA is eluted with 400 μl. TE and 1M NaCl and then ethanol precipitated. The DNA is resuspended in 20 μl. TE for subsequent ligation.

B. Ligation and construction of *E. coli* K12 SF8/pKC467

About 3 μl. (~1.5 μg.) of the isolated DNA are

*Promega Biotec, 2800 S. Fish Hatchery Road, Madison, WI 53711.

self-ligated and used to transform *E. coli* K12 SF8 in substantial accordance with the teaching of Example 2C. The identity of the desired transformants is conventionally confirmed by initially selecting for $Am^R$ phenotype and then replicating those $Am^R$ colonies to select for neomycin resistant colonies. The resultant *E. coli* K12 SF8/pKC467 transformants are conventionally cultured for subsequent production and isolation of cosmid pKC467.

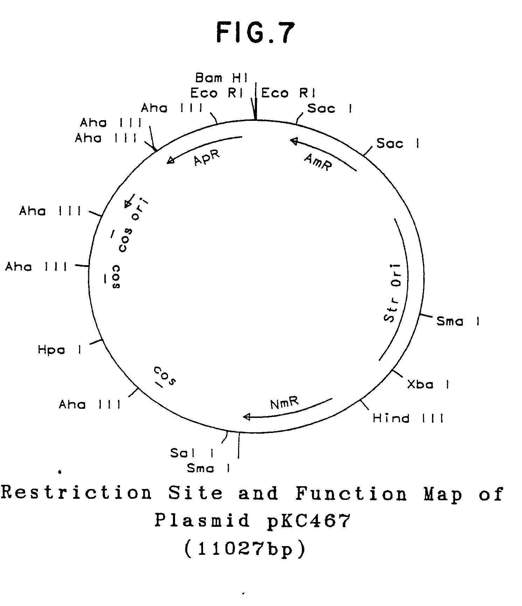

This *XbaI* deletion results in the generation of a low-copy number *Streptomyces* vector. Low-copy number vectors are advantageous in that recipient host strains are not likely to be damaged by possible high-level expression of physiologically active gene products. A restriction site and function map of cosmid pKC467 is presented in Figure 7 of the accompanying drawings.

Example 10
Construction of *Streptomyces ambofaciens*/pKC462 and *S. ambofaciens*/pKC467

About 1 μg. of DNA from Example 5 and 200 μl. of protoplasts of *Streptomyces ambofaciens* (NRRL 2420) were mixed in substantial accordance with the teaching of Example 7. The identity of the desired transformants was conventionally confirmed by initially selecting for $Am^R$ phenotype and then replicating those $Am^R$ colonies to select for neomycin resistant colonies. The resultant *S. ambofaciens*/pKC462 apramycin resistant and neomycin resistant colonies were isolated according to known procedures, cultured, and then conventionally identified by restriction enzyme and agarose gel electrophoretic analysis of their constitutive cosmids (Maniatis et al 1982).

*Streptomyces ambofaciens*/pKC467 can be constructed as taught above by substituting the pKC467 DNA from Example 9 for the pKC462 DNA.

Example 11
Culture of *E. coli* K12 SF8/pKC462A and isolation of cosmid pKC462A

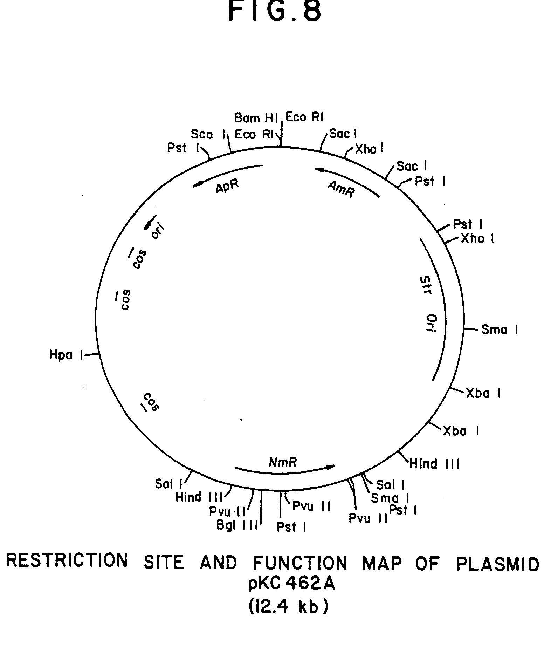

The culture of *E. coli* K12 SF8/pKC462A (NRRL B-15973) and subsequent isolation of cosmid pKC462A were carried out in substantial accordance with the teaching of Example 1. The ~0.4 mg of cosmid pKC462A DNA obtained by this procedure was suspended in 1 ml of TE buffer and stored at −20°C. A restriction site and function map of cosmid pKC462A is presented in Figure 8 of the accompanying drawings. Cosmid pKC462A can be easily distinguished from cosmid pKC462 on the basis of size because cosmid pKC462A contains approximately 0.9 bp more DNA than cosmid pKC462.

Example 12
Construction of cosmid pKC467A and *E. coli* K12 SF8/pKC467A

The construction of this low copy number *Streptomyces* vector is performed in substantial accordance with the teaching of Example 9A except that cosmid pKC462A DNA is substituted for the pKC462 DNA.

*E. coli* K12 SF8 is transformed with cosmid pKC467A DNA in substantial accordance with the teaching of Example 2C. The identity of the desired transformants can be conventionally confirmed by initially selecting for $Am^R$ phenotype and then replicating those $Am^R$ colonies to select for neomycin resistant colonies. The resultant *E. coli* K12 SF8/pKC467 transformants are conventionally cultured for subsequent production and isolation of cosmid pKC467A.

Example 13
Construction of *Streptomyces ambofaciens*/pKC462A and *S. ambofaciens*/pKC467A

About 1 µg of the DNA from Example 11 and 200 µl of protoplasts of *Streptomyces ambofaciens* (NRRL 2420) were individually mixed in substantial accordance with the teaching of Example 7. The identity of the desired transformants was conventionally confirmed by initially selecting for $Am^R$ phenotype and then replicating those $Am^R$ colonies to select for neomycin resistant colonies.

*Streptomyces ambofaciens*/pKC467A can be constructed as taught above by substituting the pKC467A DNA from Example 12 for the pKC462A DNA.

Example 14
Construction of cosmid cos111

A. Construction of intermediate plasmid pOJ107

About 25 µg of cosmid pKC462A DNA were digested in 0.5 ml of 1× buffer (150 mM NaCl, 6 mM Tris-HCl pH 7.9, 6 mM MgCl₂ and 1 mM Dithiothreitol) with 20 units of *Pst*I restriction enzyme for 3 hours at 37°C. The DNA was ethanol precipitated and collected by centrifugation. After the DNA pellet was resuspended in 100 µl of TE, the *Pst*I-digested DNA was electrophoresed on a 0.7% agarose mini gel and isolated by electrolution, using an IBI electrolution apparatus. The ~2.0 kb *Pst*I fragment was isolated by running the gel at 150V for 30 minutes, followed by ethanol precipitation and resuspension in 50 µl of TE. About 1 µl of this DNA was digested with 20 units *Bam*HI restriction enzyme at 37°C for 1 hour. The *Bam*HI-*Pst*I-digested DNA was then electrophoresed on a 0.7% agarose gel, and the desired ~1.3 kb *Bam*HI-*Pst*I restriction fragment, containing the apramycin resistance gene, was isolated and purified in substantial accordance with the teaching of Example 2B.

Plasmid pUC19 (commercially available from Pharmacia, Inc., 800 Centennial Dr., Piscataway, N.J. 08854) was similarly treated as taught above with *Bam*HI and *Pst*I restriction enzymes and the *Bam*HI-*Pst*I cut plasmid was ligated to the ~1.3 kb *Bam*HI-*Pst*I fragment of cosmid pKC462A and transformed into *E. coli* K12 SF8 in substantial accordance with the teaching of Example 2C. The desired transformants, *E. coli* K12 SF8/pOJ107, were identified by restriction enzyme analysis of their plasmid DNA and by their apramycin-resistant phenotype.

B. Construction of intermediate plasmid pOJ108

About 10 µg of plasmid pOJ107 DNA were digested in 20 µl of 1× *Eco*RI buffer with 20 units of *Eco*RI restriction enzyme. After precipitation with ethanol, these fragments were resuspended in 100 µl of *Nde*I buffer with 3 units *Nde*I restriction enzyme. The plasmid DNA mixture was incubated at 37°C for 2 hours and then the DNA was ethanol precipitated, collected by centrifugation, and dissolved in 20 µl of TE. Among the fragments generated by this double digestion is the desired *Eco*RI-*Nde*I fragment containing the *E. coli* replicon and both the ampicillin and apramycin resistance-conferring genes.

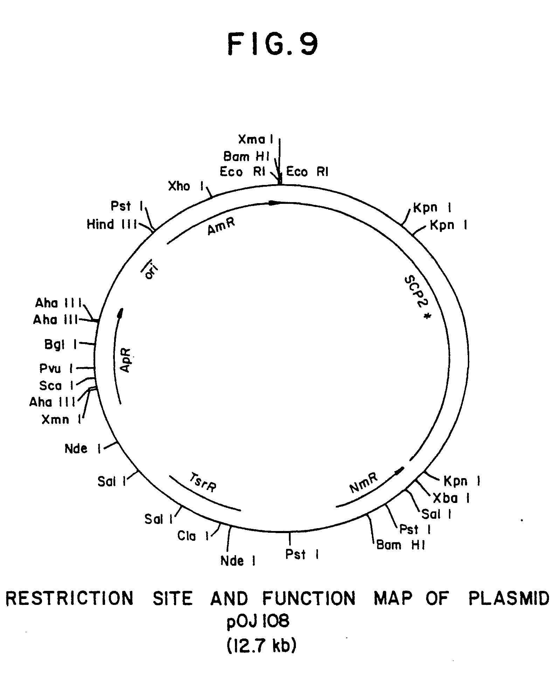

Partial *Nde*I restriction enzyme digests were performed on plasmid pHJL210 DNA (NRRL B-15824) by incubating the *Eco*RI digested pHJL210 DNA at 37°C for 8 minutes with 3 units of *Nde*I restriction enzyme. After precipitation, these fragments were cut to completion with 20 units of *Eco*RI restriction enzyme at 37°C for 2 hours. The DNA was ethanol precipitated and resuspended in 10 µl of TE. This double digest generated the desired *Eco*RI-*Nde*I fragment containing the SCP2* replicon and both the neomycin and thiostrepton resistance-conferring genes. These two plasmid digests were ligated and used to transform *E. coli* K12 SF8. The resulting transformants, *E. coli* K12 SF8/pOJ108, were identified by restriction enzyme analysis of their plasmid DNA and by their apramycin-resistant phenotype. A restriction site and function map of plasmid pOJ108 is presented in Figure 9 of the accompanying drawings.

C. Construction of intermediate plasmid pOJ111

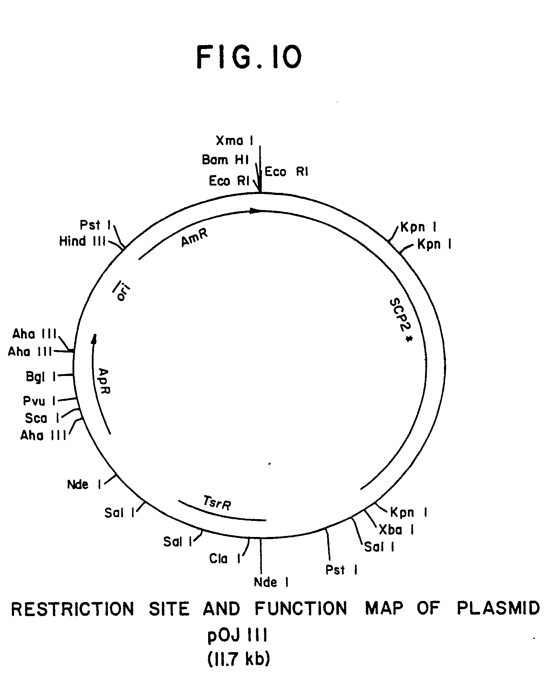

Ten µg of plasmid pOJ108 DNA was digested in 100 µl of 1× *Pst*I buffer with 50 units of *Pst*I restriction enzyme for 2 hours at 37°C. This *Pst*I digestion of plasmid pOJ108 results in an ~1.0 kb deletion, thereby removing a *Bam*HI site and inactivating the neomycin resistance-conferring gene. Upon transformation of *E. coli* K12 SF8 with the then ligated plasmid DNA, the resultant transformants were isolated and identified as plasmid pOJ111. A restriction site and function map of plasmid pOJ111 is presented in Figure 10 of the accompanying drawings.

D. Preparation of *Sca*I-*Hind*III-digested plasmid DNA

About 5 µg of plasmid pOJ111 DNA were mixed with 100 µl 1× reaction buffer (150 mM NaCl, 6 mM Tris-HCl pH 7.5, 6 mM MgCl₂ and 6 mM Dithiothreitol), and 3 µl (~50 units) of *Sca*I restriction enzyme and the resulting reaction was incubated at 37°C for 2 hours. The DNA was extracted with phenol and Sevag, precipitated with ethanol and resuspended in 20 µl of TE. The DNA was resuspended in *Hind*III buffer and then cut with 50 units of *Hind*III restriction enzyme at 37°C for 2 hours. After extraction with phenol and Sevag, the DNA was precipitated with ethanol and resuspended in 20 μl of TE.

Ten μg of plasmid pKC462A DNA were digested with HindIII and ScaI (increased to 70 units) restriction enzymes as taught above. This double digestion generates the desired HindIII-ScaI fragment containing the multiple cos sites and part of the ampicillin resistance-conferring gene. After the reaction ~3 μl (~45 units) of XhoI restriction enzyme were added to the reaction, which was then incubated at 37°C for 2 hours. This XhoI digestion serves to reduce the likelihood of parental plasmids from reappearing. The digested DNA was isolated as taught above.

E. Ligation of fragments to construct cosmid cos111 and transformation of E. coli K12 SF8

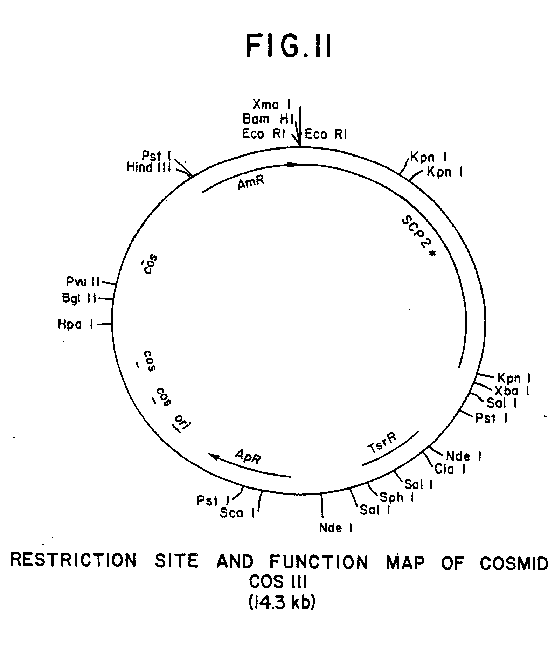

Five μl of the ScaI-HindIII restriction fragments of plasmis pOJ111 prepared in Example 14D and 5 μl of the ScaI-HindIII digest of cosmid pKC462A prepared in Example 14D were mixed together, and ligated. This ligation mix was used to transform E. coli K12 SF8. The desired E. coli K12SF7/cos111 transformants were identified by their apramycin-resistant phenotype and by restriction enzyme analysis of their cosmid DNA. Cosmid DNA was isolated from the transformants in substantial accordance with the procedure of Example 1. A restriction site and function map of cos111 is presented in Figure 11 of the accompanying drawings.

Example 15
Construction of Streptomyces lividans TK23/cos111

About 1 μg of the DNA from Example 14 and 200 μl of protoplasts of Streptomyces lividans TK23 (NRRL 15826) were individually mixed in substantial accordance with the teaching of Example 7. The identity of the desired S. lividans TK23/cos111 transformants was conventionally confirmed by initially selecting for the apramycin-resistant phenotype and then replicating those apramycin-resistant colonies to select for thiostrepton resistant colonies.

**Claims**

1. A recombinant DNA cosmid shuttle vector which comprises:
   a) a replicon that is functional in E. coli,
   b) a replicon that is functional in Streptomyces,
   c) two or more cos sites of bacteriophage lambda, and
   d) one or more DNA segments that convey resistance to at least one antibiotic when transformed into a sensitive restrictionless host cell.

2. A recombinant DNA cosmid shuttle vector as claimed in Claim 1 selected from;
   pKC420 deposited in E. coli as NRRL B-15837,
   pKC427 the restriction map of which is shown in Figure 3,
   pKC428 the restriction map of which is shown in Figure 4,
   pKC448 the restriction map of which is shown in Figure 5,
   pKC462 the restriction map of which is shown in Figure 6,
   pKC467 the restriction map of which is shown in Figure 7,
   pKC462A deposited in E. coli as NRRL B-15973,
   pKC467A prepared by XbaI deletion from pKC462A (NRRL B-15973) and
   cos111 the restriction map of which is shown in Figure 11.

3. A recombinant DNA cosmid shuttle vector as claimed in Claim 1 selected from pKC420, pKC427, pKC428, pKC448, pKC462 and pKC467.

4. Cosmid shuttle vector pKC420, pKC427, pKC428, pKC448, pKC462 or pKC467.

5. Cosmid shuttle vector pKC462A, pKC467A, or cos111.

6. A recombinant DNA cosmid shuttle vector as claimed in Claim 1 in which the E. coli replicon is selected from the replicon-containing fragments of plasmids pBR322, pBR324, pBR325 and pBR328.

7. A replicon DNA cosmid shuttle vector as claimed in Claim 1 in which the Streptomyces replicon is selected from the replicon-containing fragments of plasmids SCP2, SCP2*, SLP1 and pEL103 and pFJ265.

8. A transformed host cell selected from a restrictionless Streptomyces and E. coli, said host cell comprising a recombinant DNA cosmid shuttle vector as claimed in claim 1.

9. A restrictionless Streptomyces or E. coli host cell transformed by a recombinant DNA cosmid shuttle vector as claimed in any one of claim 1 to 6.

10. A host cell as claimed in claim 8 or 9 which is selected from Streptomyces ambofaciens, Streptomyces aureofaciens, Streptomyces cinnamonesis, Streptomyces fradiae, Streptomyces granuloruber, or Streptomyces lividans.

11. Streptomyces ambofaciens/pKC420, Streptomyces ambofaciens/pKC448, Streptomyces ambofaciens/pKC462, Streptomyces fradiae/pKC420, Streptomyces fradiae/pKC448, Streptomyces fradiae/pKC462, Streptomyces lividans/pKC420, Streptomyces lividans/pKC448, Streptomyces lividans/pKC462, Streptomyces lividans/pKC467, E. coli K12 DH1/pKC420, E. coli K12 SF8/pKC448, E. coli DH1/pKC462, Streptomyces ambofaciens/pKC462A, Streptomyces lividans/cos111, E. coli K12 SF8/pKC462A, E. coli K12 SF8/pKC467A or E. coli K12 SF8/cos111.

12. A method for constructing a genomic DNA library, which comprises:
   a) ligating a genomic DNA segment into a cosmid shuttle vector as claimed in any one of claims 1 to 7;
   b) packaging said ligated cosmid into bacteriophage lambda particles;
   c) transducing said packaged cosmid into E. coli; and
   d) transforming the recombinant cosmid into a Streptomyces host cell.

**Patentansprüche**

1. Rekombinanter DNS-Cosmid-Shuttle-Vektor, der folgene Bereiche umfaßt:

(a) ein Replikon, das in E. coli funktional ist;

(b) ein Replikon, das in Streptomyces funktional ist;

(c) zwei oder mehr cos-Bereiche des Bakteriophagen lambda; und;

(d) ein oder mehr DNS-Segmente, die Resistenz gegen wenigstens ein Antibiotikum verleihen, wenn sie in eine empfindliche restriktionsfreie Wirtszelle transformiert werden.

2. Rekombinanter DNS-Cosmid-Shuttle-Vektor nach Anspruch 1, ausgewählt unter

pKC420 hinterlegt in E. coli mit der Hinterlegungsnummer NRRL B-15837;

pKC427 dessen Restriktionskarte in Figur 3 gezeigt ist;

pKC428, dessen Restriktionskarte in Figur 4 gezeigt ist;

pKC448, dessen Restriktionskarte in Figur 5 gezeigt ist;

pKC462, dessen Restriktionskarte in Figur 6 gezeigt ist;

pKC467, dessen Restriktionskarte in Figur 7 gezeigt ist;

pKC462A, hinterlegt in E. coli mit der Hinterlegungsnummer NRRL B-15973;

pKC467A, hergestellt durch Streichung von Xba I aus pKC462A (NRRL B-15973) und

cos III, dessen Restriktionskarte in Figur 11 gezeigt ist.

3. Rekombinanter DNA-Cosmid-Shuttle-Vektor nach Anspruch 1, ausgewählt unter pKC420, pKC427, pKC428, pKC448, pKC462 und pKC467.

4. Cosmid-Shuttle-Vektor pKC420, pKC427, pKC428, pKC448, pKC462 oder pKC467.

5. Cosmid-Shuttle-Vektor pKC462A, pKC467A oder cos III.

6. Rekombinanter DNS-Cosmid-Shuttle-Vektor nach Anspruch 1, worin das E. coli-Replikon ausgewählt ist unter den ein Replikon enthaltenden Fragmenten der Plasmide pBR322, pBR324, pBR325 und pBR328.

7. Rekombinanter DNS-Cosmid-Shuttle-Vektor nach Anspruch 1, worin das Streptomyces-Replikon ausgewählt ist unter den ein Replikon enthaltenden Fragmenten der Plasmide SCP2, SCP2*, SLP1 und pEL103 und pFJ265.

8. Transformierte Wirtszelle, ausgewählt unter restriktionsfreien Zellen von Streptomyces und E. coli, wobei die Wirtszelle einen rekombinanten DNS-Cosmid-Shuttle-Vektor umfaßt, wie er in Anspruch 1 beansprucht ist.

9. Restriktionsfreie Streptomyces- oder E. coli-Wirtszelle, transformiert durch einen rekombinanten DNS-Cosmid-Shuttle-Vektor, wie er in einem der Ansprüche 1 bis 6 beansprucht ist.

10. Wirtszelle nach Anspruch 8 oder 9, ausgewählt unter Streptomyces ambofaciens, Streptomyces aureofaciens, Streptomyces cinnamonesis, Streptomyces fradiae, Streptomyces granuloruber oder Streptomyces lividans.

11. Streptomyces ambofaciens/pKC420, Streptomyces ambofaciens/pKC448, Streptomyces ambofaciens/pKC462, Streptomyces fradiae/pKC420, Streptomyces fradiae/pKC448, Streptomyces fradiae/pKC462, Streptomyces lividans/pKC420, Streptomyces lividans/pKC448, Streptomyces lividans/pKC462, Streptomyces lividans/pKC467, E. coli K12 DH1/pKC420; E. coli K12 SF8/pKC448, E. coli HD1/pKC462, Streptomyces ambofaciens/pKC462A, Streptomyces lividans/cos III, E. coli K12 SF8/pKC462, E. coli K12 SF8/pKC467A oder E. coli K12 SF8/cos III.

12. Verfahren zum Aufbau einer Genom-DNS-Bibliothek, das folgende Verfahrensschritte umfaßt:

(a) Ligieren eines Genom-DNS-Segments in einen Cosmid-Shuttle-Vektor, wie er in einem der Ansprüche 1 bis 7 beansprucht wurde,

(b) Verpacken des ligierten Cosmids in Bakteriophagen-lambda-Teilchen,

(c) Überführen des verpackten Cosmids in E. coli und

(d) Transformieren des rekombinanten Cosmids in eine Streptomyces-Wirtszelle.

**Revendications**

1. Vecteur-navette cosmidique d'ADN recombinant comprenant:

a) un réplicon qui est fonctionnel dans *E. coli*,

b) un réplicon qui est fonctionnel dans *Streptomyces*,

c) deux ou plusieurs sites cos de lambda bactériophages, et

d) un ou plusieurs segments d'ADN conférant de la résistance à au moins un antibiotique, lorsqu'ils sont transformés en cellules hôtes sensibles sans restriction.

2. Vecteur-navette cosmidique d'ADN recombinant selon la revendication 1, selectionné à partir de:

pKC420, déposé dans *E. coli*, sous le numéro matricule NRRL B-15837,

pKC427, dont la carte de restriction est représentée en figure 3,

pKC428, dont la carte de restriction est représentée en figure 4,

pKC448, dont la carte de restriction est représentée en figure 5,

pKC462, dont la carte de restriction est représentée en figure 6,

pKC467, dont la carte de restriction est représentée en figure 7,

pKC462A, déposé dans *E. coli*, sous le numero matricule NRRL B-15973,

pKC467A, préparé par suppression via XbaI, à partir de pKC462A (NRRL B-15973), et

cos111, dont la carte de restriction est représentée en figure 11.

3. Vecteur-navette cosmidique d'ADN recombinant selon la revendication 1, sélectionné à partir de pKC420, pKC427, pKC428, pKC448, pKC462 et pKC467.

4. Vecteur-cosmidique pKC420, pKC427, pKC428, pKC448, pKC462, ou pKC467.

5. Vecteur-navette cosmidique pKC462A, pKC467A ou cos111.

6. Vecteur-navette cosmidique d'ADN recombinant selon la revendication 1, dans lequel le réplicon *E. coli* est sélectionné à partir des fragments des plasmides pBR322, pBR324, pBR325, et pBR328, ces fragments contenant un réplicon.

7. Vecteur-navette cosmidique d'ADN réplicon, selon la revendication 1, dans lequel le réplicon *Streptomyces* est sélectionné à partir de fragments des plasmides SCP2, SCP2*, SLP1, et pEL103 et pFJ265, ces fragments contenant un réplicon.

8. Cellule hôte transformée, sélectionnée à partir de *Streptomyces* et *E. coli* sans restriction, cette cellule hôte comprenant un vecteur-navette cosmidique d'ADN recombinant, selon la revendication 1.

9. Cellule hôte *Streptomyces* ou *E. coli* sans restriction transformée par un vecteur-navette cosmidique d'ADN recombinant, selon l'une quelconque des revendications 1 à 6.

10. Cellule hôte selon la revendication 8 ou 9, sélectionnée à partir de *Streptomyces ambofaciens, Streptomyces aureofaciens, Streptomyces* *cinnamonesis, Streptomyces fradiae, Streptomyces granuloruber,* ou *Streptomyces lividans.*

11. *Streptomyces ambofaciens*/pKC420, *Streptomyces ambofaciens*/pKC448, *Streptomyces ambofaciens*/pKC462, *Streptomyces fradiae*/pKC420, *Streptomyces fradiae*/pKC448, *Streptomyces fradiae*/pKC462, *Streptomyces lividans*/pKC420, *Streptomyces lividans*/pKC448, *Streptomyces lividans*/pKC467, E. coli K12 DHI/pKC420, E. coli K12 SF8/pKC448, E. coli DH1/pKC462, *Streptomyces ambofaciens*/pKC462A, *Streptomyces lividans*/cos111, E. coli K12 SF8/pKC462A, E. coli K12 SF8/pKC467A ou E. coli K12 SF8/cos111.

12. Procédé de construction d'une bibliothèque d'ADN génomique, ce procédé comprenant les étapes consistant à:

a) ligaturer un segment d'ADN génomique en vecteur-navette cosmidique, selon l'une quelconque des revendications 1 à 7,

b) conditionner ce cosmide ligaturé en particules de lambda-bactériophages,

c) tranducter ce cosmide conditionne en *E. coli,* et

d) transformer le cosmide recombinant en une cellule hôte *Streptomyces.*

# FIG.I

Restriction Site and Function Map of
Cosmid pKC420
(10657bp)

# FIG.2

## CONSTRUCTION OF A GENOMIC DNA LIBRARY

VECTOR

Eco RI

Pst I

ApR  AmR

ori

pKC 420
10.6 kb

Str ori

cos

cos

cos  Bam HI

Hind III

Pvu II

Pvu II, BAP

Bam HI

INSERT DNA

> 80 kb

TOTAL STREPTOMYCES DNA

Mbo I PARTIALS, BAP

Bam HI

1.5 kb

9.1 kb  ori Am  RBam HI

LIGATE  ~ 40 kb

IN VITRO PACKAGING

TRANSDUCTION
INTO E. COLI

Eco RI

ApR  AmR  Pst I

ori  Str ori

cos  Hind III

# FIG.3

Restriction Site and Function Map of
Plasmid pKC427
(11654bp)

# FIG.4

Restriction Site and Function Map of
Plasmid pKC428
(13154bp)

## FIG. 5

Restriction Site and Function Map of
Plasmid pKC448
(11654bp)

# FIG.6

Restriction Site and Function Map of
Plasmid pKC462
(11487bp)

# FIG.7

Restriction Site and Function Map of
Plasmid pKC467
(11027bp)

# FIG.8

RESTRICTION SITE AND FUNCTION MAP OF PLASMID
pKC462A
(12.4 kb)

# FIG.9

RESTRICTION SITE AND FUNCTION MAP OF PLASMID
pOJ 108
(12.7 kb)

# FIG. 10

Xma I
Bam HI
Eco RI
Eco RI

Pst I
Hind III

AmR

ori

Kpn I
Kpn I

SCP2 *

Aha III
Aha III
Bgl I
Pvu I
Sca I
Aha III

ApR

Nde I
Sal I

TsrR

Sal I
Cla I
Nde I

Kpn I
Xba I
Sal I
Pst I

RESTRICTION SITE AND FUNCTION MAP OF PLASMID
pOJ III
(11.7 kb)

# FIG.II

RESTRICTION SITE AND FUNCTION MAP OF COSMID
COS III
(14.3 kb)